# EUROPEAN PATENT APPLICATION

(11) **EP 4 349 326 A1**
(43) Date of publication of application: **10.04.2024**
(21) Application number: 22815136.1
(22) Date of filing: 26.05.2022
(51) Int. Cl.: A61K 9/19, A61K 31/46, A61K 9/08, A61K 47/10, A61K 47/18, A61P 27/02, A61P 27/10

(54) **EYE DROP COMPOSITION FOR PREVENTION OR TREATMENT OF OPHTHALMIC DISEASES AND PREPARATION METHOD THEREFOR**

(30) Priority: 02.06.2021 CN 202110614105
(71) Applicant: Ocumension Therapeutics (Suzhou) Co., Ltd., Suzhou, Jiangsu 215124 (CN)
(72) Inventor: LIU, Ye, Suzhou City, Jiangsu 215124 (CN)
(74) Representative: Brann AB
(86) International application number: PCT/CN2022/095155
(87) International publication number: WO 2022/253090

(57) **Abstract**

An eye drop composition for the prevention or treatment of ophthalmic diseases, comprising: (a) a lyophilized preparation comprising atropine or a pharmaceutically acceptable salt thereof; and (b) a reconstituting diluent, the lyophilized preparation being separate from the reconstituting diluent.

## Description

### CROSS-REFERENCE TO RELATED APPLICATIONS

The present application claims the priority to Chinese Patent Application No. 202110614105.6, filed on June 02, 2021, which is incorporated herein by reference in its entirety.

### TECHNICAL FIELD

The present application pertains to the field of ophthalmic pharmaceutical preparations, and more specifically, generally relates to an eye drop composition for preventing or treating ophthalmic diseases and a method for preparing the same.

### BACKGROUND

In recent years, due to changes in social environments, acceleration of pace of life, popularity of video terminals, and increasing pressure from study and work, excessive eye use has caused visual fatigue, resulting in an increasing number of myopia patients, especially an increasing incidence of myopia in adolescents. A joint survey by National Health Commission and Ministry of Education of the People's Republic of China reveals that a current incidence of myopia among Chinese students is close to 60%, which is second only to Japan and ranks second in the world; and that the number of patients exceeds 60 million, ranking first in the world, and 300 thousand of people have been blinded by myopia. The incidence of myopia is also rising in Western countries.

Existing treatments mainly include health care therapy and western medicine therapy. Currently, atropine drugs are mainly used in western medicine therapy. After years of clinical research, experts and scholars from Singapore, the United States and other countries have confirmed that atropine drugs are currently the most effective drugs for preventing and treating myopia in adolescents and children. Atropine sulfate eye drops was approved by the FDA in 1960. According to USP, specifications of 1% atropine sulfate eye drops are 2 mL:20 mg, 5 mL:50 mg and 15 mL:150 mg, with indications of cycloplegia, mydriasis and amblyopia. In Singapore, 0.01% atropine sulfate eye drop is available for treatment of myopia in children and adolescents.

However, the existing atropine sulfate eye drops has the following shortcomings: (1) Atropine sulfate eye drops at an excessively high concentration have an excessively great instantaneous effect on the eyes, resulting in relatively large side effects of atropine on the eyes and non-guaranteed drug safety; while atropine sulfate eye drops at an excessively low concentration have an excessively small instantaneous effect on the eyes, and therefore, they are slow to take effect and have poor and even no efficacy, resulting in a disadvantage of a limited application scope of atropine eye drops. (2) Atropine sulfate is an ester drug, which is easy to decompose in aqueous solution, and therefore, the pharmaceutical composition is easy to hydrolyze and become ineffective during storage. When there are no elements that can stabilize hydroxide ions in the aqueous solution, atropine sulfate is prone to be affected by hydroxide ions, thereby breaking bonds and degrading to form related substances such as belladonna phenol and tropic acid. Alternatively, hydroxide ions are prone to form bonds with a chair-like structure of atropine sulfate to form related substances such as 6-hydroxyscopolamine and 7-hydroxyscopolamine. Therefore, atropine sulfate aqueous solution is an active ingredient extremely sensitive to a buffer system. A slight difference in the buffer system may cause severe degradation of atropine sulfate, thereby causing the drug to lose activity and causing the drug to be unable to exert its therapeutic effect. That is, there are stability risks in all currently commercially available eye drops.

### SUMMARY

The present disclosure is intended to overcome a problem of hydrolysis leading to inefficacy that may occur in long-term storage of atropine drug solutions in the prior art, and to provide a new eye drop composition for preventing or treating ophthalmic diseases.

To achieve the foregoing objective, an aspect of the present invention provides an eye drop composition for preventing or treating ophthalmic diseases, the eye drop composition including: (a) a lyophilized preparation including atropine or a pharmaceutically acceptable salt thereof; and (b) a reconstitution diluent, wherein the lyophilized preparation and the reconstitution diluent are separated. That is, the eye drop composition in the present disclosure is characterized in that the composition includes two independent or separate parts, i.e., the lyophilized preparation and the reconstitution diluent.

First, the lyophilized preparation includes atropine or a pharmaceutically acceptable salt thereof as an active ingredient. According to the present disclosure, the pharmaceutically acceptable salt may be a pharmaceutically acceptable acid addition salt, which refers to a salt that can retain efficacy of free base without other side effects and that is formed with inorganic or organic acid.

Specifically, inorganic acid salts may include, but are not limited to, hydrochlorides, hydrobromides, sulfates, nitrates and phosphates; and organic acid salts may include, but are not limited to, formates, acetates, 2,2-dichloroacetate, trifluoroacetate, propionate, caproate, caprylate, decanoate, undecenoate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate and naphthalenedisulfonate. These salts can be prepared in methods known in the art. In a preferred embodiment, the pharmaceutically acceptable salt may be a sulfate, that is, the pharmaceutically acceptable salt of atropine is atropine sulphate, or referred to as atropine sulfate.

Further, atropine or the pharmaceutically acceptable salt thereof is used as an active ingredient of the eye drop composition. As described above, atropine sulfate eye drops at an excessively high concentration have an excessively great instantaneous effect on the eyes, resulting in relatively large side effects of atropine on the eyes and non-guaranteed drug safety; while atropine sulfate eye drops at an excessively low concentration have an excessively small instantaneous effect on the eyes, and therefore, they are slow to take effect and have poor and even no efficacy. Therefore, in a preferred embodiment, a concentration of atropine or the pharmaceutically acceptable salt thereof may be from 0.001% to 2% (w/v), preferably from 0.01% to 0.5% (w/v), or more preferably, from 0.01% to 0.08% (w/v), for example, 0.02%, 0.03%, 0.04%, 0.05%, 0.06% or 0.07%, measured in kg/L. Unless otherwise specified, all concentration percentages in this specification are measured in w/v (kg/L), that is, the weight of the ingredient per unit volume of the corresponding preparation. In particular, for the lyophilized formulation, the percentage represents the weight of the ingredient relative to a unit volume of the lyophilized formulation prior to lyophilization.

In addition to atropine or the pharmaceutically acceptable salt thereof used as the active ingredient, the lyophilized preparation may further include one or more selected from the group consisting of a buffer salt system, a matrix agent and a stabilizer.

In a preferred embodiment, the buffer salt system may be specifically selected from one or more of a citrate buffer system and a phosphate buffer system, and is preferably the citrate buffer system; and preferably, a concentration of the buffer salt may be from 0.01% to 1%, for example, 0.05%, 0.1%, 0.2% or 0.5%. The citrate buffer system may be a citrate buffer system including anhydrous citric acid and/or sodium citrate dihydrate.

In another preferred embodiment, the matrix agent may be specifically selected from one or more of mannitol, sorbitol, sodium chloride and sucrose, and is preferably mannitol; and preferably, a concentration of the matrix agent may be from 1% to 4%, for example, 1.5%, 2%, 2.5% or 3%.

In another preferred embodiment, the stabilizer may be specifically selected from one or more of sorbitol and mannitol, and is preferably sorbitol; and preferably, a concentration of the stabilizer may be from 0.05% to 1%, for example, 0.1%, 0.2%, 0.5% or 0.8%.

Secondly, for the reconstitution diluent, the reconstitution diluent may include one or more selected from the group consisting of a buffer salt system, an osmotic pressure regulator, a chelating agent and a bacteriostatic agent.

In a preferred embodiment, the buffer salt system may be specifically selected from one or more of purified water, phosphate buffer system and citrate buffer system, and is preferably the citrate buffer system; and preferably, a concentration of the buffer salt may be from 0.01% to 1%, for example, 0.02%, 0.05%, 0.1%, 0.2% or 0.5%.

In another preferred embodiment, the osmotic pressure regulator may be specifically selected from one or more of mannitol, sodium chloride, glucose, phosphate buffer and borax, and is preferably mannitol; and preferably, a concentration of the osmotic pressure regulator may be from 1% to 4%, for example, 1.5%, 2%, 2.5% or 3%.

In another preferred embodiment, the chelating agent may be specifically disodium edetate, and preferably, a concentration of the chelating agent may be from 0.01% to 0.03%, for example, 0.015%, 0.02% or 0.025%.

In another preferred embodiment, the bacteriostatic agent may be specifically selected from one or more of benzalkonium chloride, ethylparaben, benzalkonium bromide and polyquaternium-1, and is preferably benzalkonium chloride; and preferably, a concentration of the bacteriostatic agent may be from 0.005% to 0.1%, for example, 0.01%, 0.02% or 0.05%.

It should be understood that, in addition to the active ingredient in the lyophilized preparation and reconstitution diluent in the present disclosure, the foregoing other agents used as additives may be added to the lyophilized preparation and/or reconstitution diluent separately, optionally or interchangeably according to the actual situation and the general knowledge of those skilled in the art. In addition to the ingredients or compounds specifically mentioned above, the eye drop composition in the present disclosure may also include other pharmaceutically acceptable excipients, vectors or diluents, and the like according to actual needs.

For the pH of the eye drop composition in the present disclosure, the lyophilized preparation and the reconstitution diluent can independently have a pH of from 5.0 to 6.4, and more preferably, the pH may be about 5.5, so that the eye drop composition in the present disclosure has a suitable pH value.

Another aspect of the present invention further provides a method for preparing the eye drop composition, the method specifically includes steps of: (a) contacting the buffer salt system in the lyophilized preparation with atropine or a pharmaceutically acceptable salt thereof, and optionally, one or more of a matrix agent and a stabilizer, and lyophilizing to obtain the lyophilized preparation; and (b) contacting the buffer salt system in the reconstitution diluent with one or more of an osmotic pressure regulator, a chelating agent and a bacteriostatic agent optionally to obtain the reconstitution diluent.

More specifically, step (a) may for example include: (1) weighing and adding a buffer salt into a special container, and stirring a resulting mixture to prepare a solution, for example, at a concentration from 10 mM to 25 mM; (2) adding mannitol and sorbitol sequentially, and after dissolution, adding atropine sulfate, adjusting the pH (for example, to a range from 5.0 to 6.4) and diluting the resulting mixture to a volume; (3) sterilization and filtration; (4) filling; (5) lyophilization, and so on. Step (b) may for example include: (1) weighing and adding a buffer salt into a special container, and stirring a resulting mixture to prepare a solution, for example, at a concentration from 10 mM to 25 mM); (2) adding mannitol, disodium edetate, and benzalkonium chloride solution sequentially and stirring a resulting mixture until complete dissolution, adjusting the pH (for example, to a range from 5.0 to 6.4) and diluting the resulting mixture to a volume; (3) sterilization and filtration; (4) filling, and so on. To adjust the pH, acid (HCl) solution or alkaline (NaOH) solution can be added; and the solution can be diluted to a preset volume by adding water, for example, sterile water for injection.

Another aspect of the present invention further provides a kit for preventing or treating ophthalmic diseases, the kit including: (a) a lyophilized preparation including atropine or a pharmaceutically acceptable salt thereof; (b) a reconstitution diluent; and optionally, (c) instructions for use, wherein the lyophilized preparation and the reconstitution diluent are separated.

Another aspect of the present invention further provides use of an effective dose of the foregoing eye drop composition in preparation of eye drops for treating ophthalmic diseases, wherein the preparation includes mixing the lyophilized preparation with the reconstitution diluent.

More specifically, in an embodiment of the present invention, the ophthalmic disease may be selected from the group consisting of cycloplegia, mydriasis, amblyopia, myopia or a combination thereof, especially myopia in children and adolescents.

After studies, the inventors have found that since the eye drop composition in the present disclosure includes two parts: the lyophilized preparation and the reconstitution diluent, a risk of degradation of atropine drugs in a solution form due to dehydration during long-term storage is reduced, that is, stability of a sample during long-term storage is guaranteed.

### DETAILED DESCRIPTION

The detailed description of the present disclosure is described in detail hereinafter. It should be understood that, the detailed description described here are only intended to illustrate and explain the present disclosure, rather than limiting the present disclosure.

The endpoints and any value of ranges disclosed herein are not limited to precise ranges or values, and these ranges or values should be understood to include values close to these ranges or values. For numerical ranges, combination can be made among the endpoint values of each range, between the endpoint values of each range and individual point values, and among individual point values to obtain one or more new numerical ranges, and these numerical ranges should be regarded as being specifically disclosed herein.

The embodiments of the present invention are illustrated below with the specific examples, and those skilled in the art would have readily understood other advantages and effects of the present disclosure through the disclosure of this specification, and it is obvious that the described examples are some of the examples of the present invention, rather than all of the examples. Based on the examples of the present invention, all other examples obtained by those of ordinary skills in the art without involving any inventive effort fall within the claimed scope of the present invention.

In the following examples, stability criteria shown in the following table are used to determine stability of a lyophilized preparation, a reconstitution diluent and the reconstituted eye drops, to determine a stability result of a preparation in the present disclosure.

### Stability criteria for lyophilized preparations:

| **Test items** | **Method** | Acceptable criteria ^{a} |
|---|---|---|
| Characteristic | Visual inspection | White to off-white lumps with no visible contaminants |
| pH | USP<791> | Report results |
| | | 5.9±0.5 |
| Reconstitution time | Visual inspection | No more than 30 seconds |
| Identification | Enterprise HPLC method | Retention time of a main peak of the test solution was consistent with that of the control solution |
| Osmotic pressure molar concentration | USP<785> | Report results |
| Content (atropine sulfate) | Enterprise HPLC method | 90.0% to 110.0% |
| | | 97.0% to 103.0% |
| Related substances | Enterprise HPLC method | Report results |
| Moisture content | USP<921> | Report results |
| | | No more than 1.0% |
| Sterility | USP<71> | Sterility |

### Stability criteria for diluents:

| **Test items** | **Method** | Acceptable criteria ^{a} |
|---|---|---|
| Characteristic | Visual inspection | Colorless clear solution without visible particles |
| Osmotic pressure molar concentration | USP<785> | 230 mOsm/kg to 270 mOsm/kg |
| pH | USP<791> | 5.7 to 6.1 |
| | | 5.9±0.5 |
| Content (benzalkonium chloride) | Enterprise HPLC method | 80.0% to 120.0% |
| Content (disodium edetate) | Enterprise HPLC method | 80.0% to 120.0% |
| Sterility | USP<71> | Sterility |
| Insoluble particles | USP<789> | In accordance with the criteria |

### Stability criteria for eye drops:

| **Test items** | **Method** | **Acceptable criteria** |
|---|---|---|
| Characteristic | Visual inspection | Colorless clear solution without visible particles basically |
| pH | USP<791> | 5.7 to 6.1 |
| Osmotic pressure | USP<785> | 280 mOsm/kg to 320 mOsm/kg |
| Content (atropine sulfate) | Enterprise HPLC method | 90.0% to 110.0% |
| Related substances | Enterprise HPLC method | Report results |
| Content (benzalkonium chloride) | Enterprise HPLC method | 80.0% to 120.0% |
| Content (disodium edetate) | Enterprise HPLC method | 80.0% to 120.0% |
| Insoluble particles | USP<789> | The concentration of ≥ 10 µm particles should not exceed 50 particles/mL. |
| | | The concentration of ≥ 25 µm particles should not exceed 5 particles/mL. |
| | | The concentration of ≥ 50 µm particles should not exceed 2 particles/mL. |
| Reconstitution time | Visual inspection | After 30 seconds of reconstitution, there was almost no visible foreign matter. |

### Example 1

### Formulations of the lyophilized preparations:

| **Formulation** | **Formulation R1** | **Formulation R2** |
|---|---|---|
| Buffer solution | 20 mM citrate buffer solution | 20 mM citrate buffer solution |
| Mannitol | 3% | 4% |
| Sorbitol | 0% | 1% |
| Atropine sulfate | 0.08% | 0.08% |

The lyophilized preparations were prepared according to the foregoing formulations, and stability results still met the requirements after 6 months, indicating that both the formulations could meet the stability requirements.

### Example 2

### Formulations of the reconstitution diluent

| **Formulation** | **Formulation R1** | **Formulation R2** |
|---|---|---|
| Buffer solution | Citrate buffer solution | Citrate buffer solution |
| Mannitol | 4% | 4% |
| Sorbitol | 1% | 1% |
| Atropine sulfate | 0.08 % | 0.08% |
| Reconstitution diluent | 10 mM sodium phosphate buffer solution | 10 mM citrate buffer solution |

The reconstitution diluents were prepared according to the foregoing formulations, and the pH was adjusted to 6.0. Stability data showed that both the samples in the two buffer systems had good stability during a stability period.

### Example 3

### Determination of concentrations of benzalkonium chloride in prescriptions

The concentration of benzalkonium chloride (BAK) in atropine sulfate eye drops was verified via antimicrobial efficacy testing (AET). The lyophilized preparation was reconstituted with 8 mL of reconstituted diluent including BAK at concentrations of 100 ppm, 80 ppm, 50 ppm, 25 ppm, or 0 ppm to obtain atropine sulfate eye drops (0.01%) including benzalkonium chloride at different concentrations. The results showed that for all tested concentrations except 0 ppm, results of the antibacterial efficacy testing met the criteria.

| **Concentrations of BAK** | **United States Pharmacopeia Method** | **European Pharmacopoeia Method, Criteria A&B** | **Chinese Pharmacopoeia Method, Criteria A&B** |
|---|---|---|---|
| 0 ppm | Fail | Fail | Fail |
| 25 ppm | Pass | Pass | Pass |
| 50 ppm | Pass | Pass | Pass |
| 80 ppm | Pass | Pass | Pass |
| 100 ppm | Pass | Pass | Pass |

According to the criteria in the Chinese Pharmacopoeia, when the concentration of benzalkonium chloride in the prescription was greater than 70 ppm, it could be more effectively ensured that the criteria requirements of antibacterial efficacy "A" were met during long-term storage.

### Example 4

### Eye drop composition

1. Formulations of the lyophilized preparation and the reconstitution diluent are shown in following table:

| **Lyophilized preparation** | |
|---|---|
| Atropine sulfate | 0.08% |
| Anhydrous citric acid | 0.074% |
| Sodium citrate dihydrate | 0.49% |
| Mannitol | 4% |
| Sorbitol | 1% |
| 1.0M HCl or 1.0M NaOH | Proper dose, to adjust the pH to 5.9 |
| Sterile water for injection | Quantitatively diluted to a volume |

| **Reconstitution diluent** | |
|---|---|
| Citric Acid Monohydrate | 0.037% |
| Sodium citrate dihydrate | 0.243% |
| Mannitol | 3.8 % |
| Disodium edetate | 0.025% |
| Benzalkonium chloride | 0.01% |
| 1.0M HCl or 1.0M NaOH | Proper dose, to adjust the pH to 5.9 |
| Water for injection | Quantitatively diluted to a volume |

### 2. Process Flow

Preparation of the lyophilized preparation (1) The buffer salt was weighed and added into a suitable container, water for injection was add, a resulting mixture was stirred for 20 minutes until complete dissolution, pH was adjusted, and water for injection was added to dilute the resulting mixture to a volume. (2) A certain amount of buffer was weighed and added into the preparation container, other excipients were added in sequence, and after visual inspection of the solution, atropine sulfate was added, a resulting mixture was stirred for dissolution, and the pH of the solution was adjusted to a target value.

Preparation of the reconstitution diluent (1) The buffer salt was weighed and added into a suitable container, water for injection was add, and a resulting mixture was stirred until complete dissolution; (2) then other excipients were added in sequence, and a resulting mixture was stirred for dissolution; and (3) the pH of the solution was adjusted, and the resulting mixture was diluted to a volume.

### 3. Stability Results

According to the foregoing stability criteria and test results, the lyophilized preparation and the reconstitution diluent were stored at 25°C±2°C/60%RH±5%RH for 12 months, or stored at 40°C±2°C/75%RH±5%RH for 6 months, and their stability results were good. After reconstitution, the eye drops were stored at 25°C/60%RH for 8 weeks, and their stability results were good.

As can be seen from the results of the foregoing Examples 1 to 4, the eye drop composition provided in the present disclosure had good stability. That is, preparing separate atropine eye drops into the lyophilized preparation could effectively resolve the problem of poor stability of the atropine eye drops as a solution.

The preferred embodiments of the present disclosure have been described in detail above. However, the present disclosure is not limited to the specific details in the above embodiments. Within the scope of the technical concept of the present disclosure, various simple modifications can be made to the technical solution of the present disclosure, all of which fall within the claimed scope of the present disclosure.

Furthermore, it should be noted that various specific technical features described in the above specific embodiments can be combined in any suitable way without contradiction. In order to avoid unnecessary repetition, the present disclosure will not explain various possible combinations separately.

Furthermore, any combination can be made among various embodiments of the present disclosure, as long as it does not violate the concept of the present disclosure, and it should also be regarded as the disclosure of the present disclosure.

## Claims

1. An eye drop composition for preventing or treating an ophthalmic disease, **characterized in that** the eye drop composition comprises:
(a) a lyophilized preparation comprising atropine or a pharmaceutically acceptable salt thereof; and
(b) a reconstitution diluent,
wherein the lyophilized preparation and the reconstitution diluent are separated.

2. The eye drop composition of claim 1, wherein the pharmaceutically acceptable salt of atropine is atropine sulfate.

3. The eye drop composition of claim 1, wherein a concentration of atropine or the pharmaceutically acceptable salt thereof is in a range from 0.001% to 2%, preferably from 0.01% to 0.5%, or more preferably from 0.01% to 0.08%.

4. The eye drop composition of claim 1, wherein a pH of the eye drop composition is in a range from 5.0 to 6.4.

5. The eye drop composition of claim 1, wherein the lyophilized preparation further comprises one or more selected from the group consisting of a buffer salt system, a matrix agent and a stabilizer.

6. The eye drop composition of claim 5, wherein the buffer salt system is selected from one or more of a citrate buffer system and a phosphate buffer system, and is preferably the citrate buffer system; and preferably, a concentration of the buffer salt is from 0.01% to 1%.

7. The eye drop composition of claim 5, wherein the matrix agent is selected from one or more of mannitol, sorbitol, sodium chloride and sucrose, and is preferably mannitol; and preferably, a concentration of the matrix agent is from 1% to 4%.

8. The eye drop composition of claim 5, wherein the stabilizer is selected from one or more of sorbitol and mannitol, and is preferably sorbitol; and preferably, a concentration of the stabilizer is from 0.05% to 1%.

9. The eye drop composition of claim 1, wherein the reconstitution diluent comprises one or more selected from the group consisting of a buffer salt system, an osmotic pressure regulator, a chelating agent and a bacteriostatic agent.

10. The eye drop composition of claim 9, wherein the buffer salt system is selected from one or more of purified water, phosphate buffer system and citrate buffer system, and is preferably the citrate buffer system; and preferably, a concentration of the buffer salt is from 0.01% to 1%.

11. The eye drop composition of claim 9, wherein the osmotic pressure regulator is selected from one or more of mannitol, sodium chloride, glucose, phosphate buffer and borax, and is preferably mannitol; and preferably, a concentration of the osmotic pressure regulator is from 1% to 4%.

12. The eye drop composition of claim 9, wherein the chelating agent is disodium edetate, and preferably, a concentration of the chelating agent is from 0.01% to 0.03%.

13. The eye drop composition of claim 9, wherein the bacteriostatic agent is selected from one or more of benzalkonium chloride, ethylparaben, benzalkonium bromide and polyquaternium-1, and is preferably benzalkonium chloride; and preferably, a concentration of the bacteriostatic agent is from 0.005% to 0.1%.

14. A method for preparing the eye drop composition of any one of claims 1 to 13, **characterized in that** the method comprises steps of:
(a) contacting the buffer salt system in the lyophilized preparation with atropine or a pharmaceutically acceptable salt thereof, and optionally, one or more of a matrix agent and a stabilizer, and lyophilizing to obtain the lyophilized preparation; and
(b) contacting the buffer salt system in the reconstitution diluent with one or more of an osmotic pressure regulator, a chelating agent and a bacteriostatic agent optionally to obtain the reconstitution diluent.

15. The method of claim 14, wherein step (a) and step (b) each independently comprise: adjusting the pH of the mixed solution to a range from 5.0 to 6.4 after the contacting is completed.

16. A kit for preventing or treating an ophthalmic disease, **characterized in that** the kit comprises:
(a) a lyophilized preparation comprising atropine or a pharmaceutically acceptable salt thereof;
(b) a reconstitution diluent; and
optionally, (c) instructions for use,
wherein the lyophilized preparation and the reconstitution diluent are separated.

17. Use of an effective amount of the eye drop composition of any one of claims 1 to 13 in preparation of an eye drop for treating an ophthalmic disease, **characterized in that** the preparation comprises mixing the lyophilized preparation with the reconstitution diluent.

18. The use of claim 17, wherein the ophthalmic disease is selected from the group consisting of cycloplegia, mydriasis, amblyopia, myopia or a combination thereof, especially myopia in children and adolescents.
